# EUROPÄISCHE PATENTSCHRIFT

(11) **EP 0 347 676 B1**
(45) Veröffentlichungstag und Bekanntmachung des Hinweises auf die Patenterteilung: **15.09.1993**
(21) Anmeldenummer: 89110523.1
(22) Anmeldetag: 10.06.1989
(51) Int. Cl.: C07D 231/16, C07D 231/56

(54) **Verfahren zur Herstellung von N-Hydroxypyrazolen**
Process for the preparation of N-hydroxypyrazoles
Procédé de préparation de pyrazoles N-hydroxyliques

(30) Priorität: 18.06.1988 DE 3820738
(43) Veröffentlichungstag der Anmeldung: 27.12.1989
(73) Patentinhaber: BASF Aktiengesellschaft, 67063 Ludwigshafen (DE)
(72) Erfinder: Baus, Ulf, Dr., D-6915 Dossenheim (DE); Reuther, Wolfgang, Dr., D-6900 Heidelberg (DE); Fikentscher, Rolf, Dr., D-6700 Ludwigshafen (DE)

(56) Entgegenhaltungen:
- EP-A- 0 215 380
- EP-A- 0 249 977
- EP-A- 0 347 689
- DE-A- 3 031 385
- DE-A- 3 205 456
- DE-A- 3 409 317
- HOUBEN-WEYL, "Methoden der Organischen Chemie", Band X/1, 1971, Seiten 1135-1137, Teil 1, Georg Thieme Verlag, Stuttgart, DE
- JOURNAL OF ORGANIC CHEMISTRY, Band 34, Nr. 1, Januar 1969, Seiten 187-194, Easton, US; J.P. FREEMAN et al.: "The nitrosation of alpha,beta-unsaturated oximes. IV. The synthesis and structure of 3,4-diazacyclopentadienone derivatives"

## Beschreibung

Die Erfindung betrifft die Herstellung von N-Hydroxypyrazol und seinen Derivaten aus den entsprechenden Pyrazolen.

N-Hydroxypyrazol und seine Derivate sind wichtige Zwischenprodukte für die Herstellung von Substanzen mit breitem biologischen Wirkungsspektrum. Beispielsweise wird in DE 3 409 317 die Herstellung von wertvollen Nitrifikationsinhibitoren für Ammoniumstickstoff aus N-Hydroxypyrazolen beschrieben. Weiterhin wird in DE 35 32 880 die Herstellung von 1,4-disubstituierten Derivaten des N-Hydroxypyrazols als Verbindungen mit selektiver Wirkung auf Histamin-H₂-Rezeptoren beschrieben. Es hat daher nicht an Versuchen gefehlt ein vorteilhaftes Verfahren zur Herstellung von N-Hydroxypyrazolen zu finden.

Gemäß der DE-OS 3 031 385 wird N-Hydroxypyrazol durch eine mehrstufige Synthese aus den Bausteinen Azodicarbonsäureester, Cyclopentadien, Nitriloxid und einem Peroxid hergestellt. Nachteilig an diesem Verfahren ist die mehrstufige Reaktionsführung, die geringe Ausbeute sowie die Tatsache, daß das N-Hydroxypyrazol im Gemisch mit Isoxazolen anfällt.

Aus DE-OS 3 205 456 ist ein Verfahren zur Herstellung von halogenierten N-Hydroxypyrazolen bekannt, mit dessen Hilfe die gemäß dem Verfahren von DE-OS 3 031 385 hergestellten N-Hydroxypyrazole halogeniert werden können.

Kernsubstituierte N-Hydroxypyrazole können beispielsweise gemäß J. Org. Chem. 34 (1969), Seiten 187-94 durch Nitrosierung entsprechend substituierter α,β-ungesättigter Oxime und anschließende Reduktion der erhaltenen 3,4-Diazacyclopentadienondioxide hergestellt werden. Nachteilig an diesem Verfahren sind die mehrstufige Verfahrensführung sowie die Tatsache, daß nur kernsubstituierte N-Hydroxypyrazole hergestellt werden können.

Es war daher die Aufgabe der Erfindung, ein einfaches und wirtschaftliches Verfahren zu finden, das die Herstellung sowohl von N-Hydroxypyrazol selbst als auch von kernsubstituierten N-Hydroxypyrazolen ermöglicht.

Es wurde nun überraschenderweise gefunden, daß man N-Hydroxypyrazol selbst sowie kernsubstituierte H-Hydroxypyrazole auf einfache Weise erhält, wenn man die entsprechenden Pyrazole in ihre Alkalisalze überführt und diese unter definierten Reaktionsbedingungen mit einem Diacylperoxid, vorzugsweise mit Dibenzoylperoxid umsetzt.

Es war zwar bekannt, daß sich bei der Umsetzung von primären oder sekundären aliphatischen Aminen mit Wasserstoffperoxid oder seinen Acylderivaten wie Dibenzoylperoxid in Verbindung mit Katalysatoren und anschließende Verseifung der dabei gebildeten O-Benzoyl-N-hydroxylamine Hydroxylamine herstellen lassen (vgl. Houben-Weyl, Methoden der organischen Chemie, Bd. 10/1, Thieme-Verlag, 1971, Seiten 1135-1137), jedoch wird in loc. cit. diese Reaktion ausdrücklich auf aliphatische Amine beschränkt. Gegenstand der Erfindung ist dementsprechend ein Verfahren zur Herstellung von N-Hydroxypyrazolen der allgemeinen Formel I 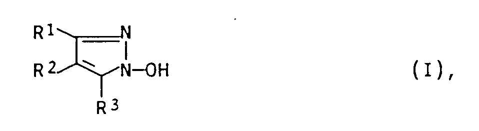
in der
- R¹, R² und R³: gleich oder verschieden sein können und Wasserstoff oder einen Alkyl, Cycloalkyl- oder Arylrest oder Halogen bedeuten können
oder aber die Substituenten
- R¹ und R² oder R² und R³: zusammen mit den diese Reste tragenden Kohlenstoffatomen einen aromatischen oder nichtaromatischen Ring mit 5 oder 6 C-Atomen, bedeuten
das dadurch gekennzeichnet ist, daß man
A) Pyrazole der allgemeinen Formel II 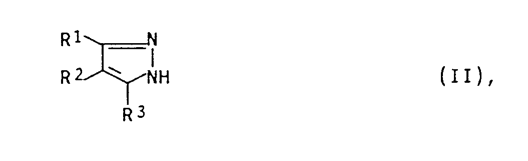 in der
   die Reste R¹, R² und R³ die in Formel I angegebene Bedeutung haben, in an sich bekannter Weise mit einem Alkalihydroxid, Alkalihydrid oder Alkalicarbonat bei Temperaturen von 0 bis 50°C in ihre Metallsalze der allgemeinen Formel III 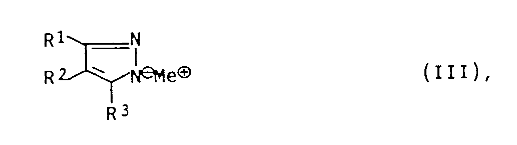 in der
   die Reste R¹, R² und R³ die in Formel I genannte Bedeutung haben, und
   Me^{⊕} ein Kation der Alkalimetalle bedeutet, überführt und
B) die erhaltenen Metallsalze der allgemeinen Formel III so mit Diacylperoxiden, vorzugsweise mit Dibenzoylperoxid, umsetzt, daß die Reaktionstemperatur zwischen 0 und 60°C liegt.

Als Pyrazole der allgemeinen Formel II kommen beispielsweise in Betracht: Indazol, 3-Phenyl-pyrazol, 4-Phenyl-pyrazol, 3,5-Diphenyl-pyrazol, 3,4,5-Triphenyl-pyrazol, 3,5-Diphenyl-4-methyl-pyrazol, 4,5-Dimethyl-pyrazol und 3,4,5-Trimethyl-pyrazol.

Bevorzugte Pyrazole der Formel II sind beispielsweise Pyrazol selbst, 4-Chlor-pyrazol, 4-Brom-pyrazol, 4-Jod-pyrazol und 3,5-Dimethyl-pyrazol.

Die Überführung der Pyrazole in ihre Metallsalze der allgemeinen Formel III erfolgt in an sich bekannten Weise durch Umsetzen der Pyrazole in einem inerten Lösungsmittel mit einem Alkalihydroxid, Alkalihydrid oder Alkalicarbonat bei Temperaturen von 0 bis 50°C.

Als Diacylperoxide kommen symmetrische aliphatische oder aromatische Diacylperoxide in Betracht. Genannt seien beispielsweise Dilaurylperoxid, Didecanoylperoxid, Bis-(3,5,5-trimethyl-hexanoyl)-peroxid, Bis-(3-chlorbenzoyl)-peroxid und Bis-(2,4-dichlorbenzoyl)-peroxid. Die symmetrischen Diacylperoxide kann man auf einfache Weise durch Umsetzen von H₂O₂ mit Carbonsäurechloriden in Gegenwart von Alkalihydroxiden oder Alkalicarbonaten herstellen. Aber auch unsymmetrische Diacylperoxide, wie beispielsweise Phenylacetylperoxid, können für die erfindungsgemäße Umsetzung eingesetzt werden. Man erhält sie durch Umsetzen von Percarbonsäuren mit einem Carbonsäurechlorid in Gegenwart von Alkalihydroxiden oder -carbonaten. Mit besonderem Vorteil verwendet man Dibenzoylperoxid.

Das erfindungsgemäße Verfahren kann auf verschiedene Weise vorteilhaft durchgeführt werden. Eine Möglichkeit besteht darin, daß man die Pyrazole der Formel II in einem inerten organischen Lösungsmittel in an sich bekannter Weise in ihre Metallsalze der Formel III überführt und anschließend zu der erhaltenen Lösung bzw. Suspension der Metallsalze der Formel III eine Lösung von möglichst trockenem Dibenzoylperoxid in einem inerten Lösungsmittel in der Weise zugibt, daß die Reaktionstemperatur zwischen 0 und 60°C, vorzugsweise zwischen 5 und 20°C, liegt.

Das für diese Umsetzung benötigte Dibenzoylperoxid kann auf einfache Weise durch Umsetzen von H₂O₂ mit Benzoylchlorid in Gegenwart von Alkalihydroxiden oder Alkalicarbonaten hergestellt werden.

Es kann in trockenem, aber auch in feuchtem Zustand eingesetzt werden; die besten Ausbeuten erzielt man, wenn man mit einer Lösung von trockenem Dibenzoylperoxid arbeitet.

Als Lösungsmittel für diese Umsetzung sind aliphatische Ether, wie Diethylether, Diisopropylether, Diethylenglykoldimethylether; aliphatische Kohlenwasserstoffe, wie Pentan, Hexan und Cyclohexan; cyclische Ether, wie Tetrahydrofuran (THF) und Dioxan sowie aromatische Kohlenwasserstoffe, wie Benzol und Toluol geeignet.

Mit besonderem Vorteil verwendet man Diethylether, THF und Toluol.

Das stöchiometrische Verhältnis von III zu Dibenzoylperoxid kann hierbei zwischen 1:1 und 5:1 variieren, vorzugsweise wird mit einem Überschuß an dem Pyrazolsalz III gearbeitet, d.h. etwa im Verhältnis 2:1 bis 3:1.

Im allgemeinen ist die Umsetzung nach Beendigung der Zugabe abgeschlossen. In manchen Fällen ist es jedoch von Vorteil das Reaktionsgemisch noch eine kurze Zeit bei Raumtemperatur nachrühren zu lassen.

Es ist jedoch nicht unbedingt nötig, daß man mit völlig trockenem Dibenzoylperoxid arbeitet. Sehr geringe Mengen Feuchtigkeit oder aber die Anwesenheit von etwa 1 Äquivalent Wasser pro Äquivalent Pyrazol, wie es bei der Herstellung der Metallsalze der Formel III aus dem Pyrazol und Alkalihydroxiden gebildet wird, vermindern die Ausbeuten an Hydroxypyrazolen nur unwesentlich.

Man kann jedoch auch in Gegenwart von größeren Mengen an Wasser sehr vorteilhaft arbeiten, wenn man die Umsetzung in einem mit Wasser nicht oder nur wenig mischbaren Lösungsmittel vornimmt und dem Reaktionsgemisch katalytische Mengen eines Phasentransferkatalysators, wie beispielsweise einem quartären Ammoniumsalz zusetzt.

Gegenstand der Erfindung ist daher auch ein Verfahren zur Herstellung von N-Hydroxypyrazolen der allgemeinen Formel I, das dadurch gekennzeichnet ist, daß man
A) die Pyrazole der allgemeinen Formel II in Wasser in an sich bekannter Weise mit einem Alkalihydroxid oder Alkalicarbonat in Lösungen oder Suspensionen ihrer Metallsalze der allgemeinen Formel III überführt und
B) die erhaltenen Metallsalze der allgemeinen Formel III bzw. ihre Lösungen oder Suspensionen in Wasser in einem 2-Phasensystem aus Wasser und einem inerten, mit Wasser nicht oder nur wenig mischbaren, organischen Lösungsmittel in Gegenwart eines geeigneten Phasentransferkatalysators mit Dibenzoylperoxid umsetzt.

Diese Variante des erfindungsgemäßen Verfahrens hat den großen Vorteil, daß man das in fester Form handelsübliche, mit einer größeren Menge Wasser (meist 25 % Wasser) phlegmatisierte Dibenzoylperoxid verwenden kann, wodurch besondere Vorsichtsmaßnahmen, die beim Arbeiten mit Lösungen von weitgehend trockenem Dibenzoylperoxid unerläßlich sind, entfallen können.

Für das erfindungsgemäße Verfahren lassen sich zahlreiche Phasentransfer-Katalysatoren (allg. Übersicht vgl. V. Dehmlow, Angew. Chemie, 89 (1977), Seite 521 bis 533) verwenden. Besonders bevorzugte Phasentransferkatalysatoren (PTK) sind:
1. Tetraalkylammoniumsalze der allgemeinen Formel IV 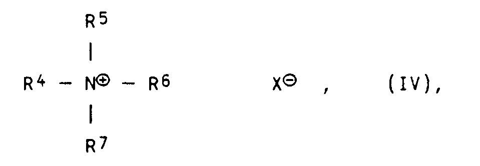 in der
   R⁴, R⁵, R⁶ und R⁷ untereinander gleich oder verschieden sein können und für Alkylreste mit 1 bis 22 Kohlenstoffatomen oder für funktionelle Gruppen, wie Hydroxyl-, Carbonamid- oder Ethergruppen enthaltende Alkylreste mit bis zu 25 Kohlenstoffatomen wie Methyl-, Ethyl-, (Iso)propyl-, Butyl-, Octyl-, Dodecyl-, C₁₆H₃₃-, Hydroxy(iso)propyl- oder 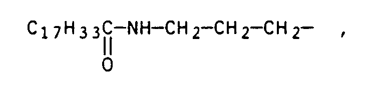 sowie für Phenylreste oder phenylsubstituierte Alkylreste (wie z.B. Benzyl-) mit bis zu 20 Kohlenstoffatomen stehen können,
   und X^{⊖} für ein Anion einer Säure wie J⁻, Cl⁻, Br⁻, (HSO₄)⁻, (CN)⁻, (BF₄)⁻ oder OH⁻ steht; insbesondere das sehr preiswerte Trimethylbenzylammoniumchlorid, das in Form seiner 50 %igen wäßrigen Lösung eingesetzt werden kann, sowie Tricaprylmethylammoniumchlorid; und
2. Tetraalkylphosphoniumsalze der allgemeinen Formel V 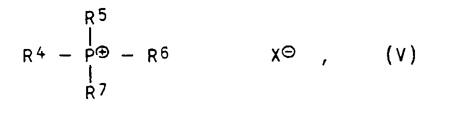 worin R⁴, R⁵, R⁶, R⁷ sowie X^{⊖} die für die Formel (IV) angegebene Bedeutung haben können, insbesondere das Tri-n-octyl-methylphosphoniumiodid.

Weiterhin geeignet sind auch Gemische der oben genannten PTK sowie Trägerfixierte PTK.

Die PTK werden für das erfindungsgemäße Verfahren in Mengen von 0,1 bis 1, vorzugsweise 0,3 bis 0,5 Mol pro Mol Pyrazol eingesetzt.

Das stöchiometrische Verhältnis zwischen dem Metallsalz III und Dibenzoylperoxid wählt man bei dieser Variante mit Vorteil etwas höher. Es kann variieren zwischen 1:1 und 10:1, vorzugsweise zwischen 3:1 und 6:1.

Folgende Durchführungsweisen für das erfindungsgemäße Verfahren haben sich besonders bewährt:
1) Man gibt handelsübliches, mit einer größeren Menge Wasser phlegmatisiertes Dibenzoylperoxid in festem Zustand bei Raumtemperatur zu einem 2-Phasensystem, welches aus Wasser, einem Alkalihydroxid, einem Pyrazol der Formel II, einem Phasentransferkatalysator und einem mit Wasser nicht oder nur wenig mischbaren inerten Lösungsmittel hergestellt wurde.
2) Man legt handelsübliches Dibenzoylperoxid in einem 2-Phasensystem, bestehend aus Wasser, einem Pyrazol der Formel II, einem Phasentransferkatalysator und einem mit Wasser nicht oder nur wenig mischbaren inerten organischen Lösungsmittel vor und ermöglicht die Umsetzung durch Zugabe eines Alkalihydroxids.

Man kann bei der ersten Durchführungsweise aber auch das einzusetzende Dibenzoylperoxid in einem Lösungsmittel lösen und die Lösung - ggf. nach Abtrennen von abgeschiedenem Wasser - zu dem 2-Phasensystem zudosieren.

Als Alkalihydroxid verwendet man im allgemeinen KOH oder NaOH. Es kann in Pulverform oder als wäßrige Lösung eingesetzt werden. Man verwendet es im allgemeinen in Mengen von 1 bis 10 Mol, vorzugsweise 1 bis 1,5 Mol pro Mol Pyrazol.

Das Wasser kann im Reaktionsgemisch in Mengen von 1 bis >100 Mol pro Mol Pyrazol enthalten sein. Als mit Wasser nicht oder nur wenig mischbare Lösungsmittel seien beispielsweise aromatische Kohlenwasserstoffe, wie Benzol oder Toluol, halogenierte Kohlenwasserstoffe, wie Methylenchlorid, Chloroform oder Chlorbenzol oder aliphatische Kohlenwasserstoffe, wie Cyclohexan genannt.

Zur Aufarbeitung des Reaktionsgemisches versetzt man dieses im allgemeinen mit Wasser und trennt nach gutem Durchmischen die wäßrige Phase ab. Die bei der Reaktion gebildete Benzoesäure kann dann z.B. durch Ansäuern der wäßrigen Phase mit einer Säure, wie Schwefelsäure, ausgefällt und extrahiert oder durch Filtration abgetrennt werden.

Anschließend können das gewünschte N-Hydroxypyrazol und überschüssiges Pyrazol durch Extraktion bei schwach saurem pH-Wert mit einem geeigneten Lösungsmittel, wie beispielsweise Essigsäureethylester, in die organische Phase überführt und nach destillativem Entfernen des Lösungsmittels, destillativ getrennt werden. Hierzu wurde die Mischung schwach basisch gestellt und in einem Rotationsverdampfer unter stark vermindertem Druck (>7 mbar) eingeengt. Der feste Rückstand wird schwach angesäuert und beispielsweise mit Essigsäureethylester extrahiert. Die Essigesterphase wird getrocknet und eingeengt. Der verbleibenden Rückstand kann aus Cyclohexan umkristallisiert werden. Auf diese Weise wird das im Überschuß eingesetzte und nicht verbrauchte Pyrazol zurückgewonnen und man erhält sehr reines N-Hydroxypyrazol.

Mit Hilfe des erfindungsgemäßen Verfahrens können die als Zwischenprodukte für zahlreiche Wirkstoffe begehrten N-Hydroxypyrazole auf recht einfache und wirtschaftliche Weise in guten Ausbeuten und in reiner Form erhalten werden.

Das erfindungsgemäße Verfahren sei an folgenden Beispielen erläutert:

### Beispiel 1

a) 20,4 g (0,3 Mol) Pyrazol wurden in 500 ml Tetrahydrofuran (THF) gelöst und bei Raumtemperatur (RT) in kleinen Portionen mit 9 g (0,3 Mol) einer 80 gew.%igen Suspension von NaH in Paraffinöl versetzt. Nach beendeter Wasserstoffentwicklung wurde die Mischung auf 5°C abgekühlt und mit 24,2 g (0,1 Mol) Dibenzoylperoxid (trocken), gelöst in 500 ml THF, so versetzt, daß die Temperatur der Reaktionsmischung nicht über 25°C stieg.
   Nach beendeter Zugabe wurde noch 10 Minuten (min) gerührt und die Reaktionsmischung mit Eiswasser und Petrolether (Kp = 30 bis 60°C) versetzt und geschüttelt. Die wäßrige Phase wurde abgetrennt und durch Zusatz von Schwefelsäure angesäuert. Nach mehrfacher Extraktion mit Cyclohexan wurde die wäßrige Phase durch Zugabe von KOH-Lösung, auf einen pH-Wert von 8 eingestellt und anschließend unter stark vermindertem Druck (<7 mbar/Wasserbad >30°C) eingedampft. Das ausgefallene Salz wurde in wenig Wasser aufgenommen, mit Schwefelsäure leicht angesäuert und mit Essigsäureethylester ausgeschüttelt. Die organische Phase wurde mit Natriumsulfat getrocknet und das Lösungsmittel im Rotationsverdampfer entfernt.
   Ausbeute: 3,95 g (entsprechend 47 % der Theorie, bezogen auf umgesetztes Pyrazol) Festpunkt: 74°C (F in Lit.: 74°C)

In analoger Weise wurden aus entsprechend substituierten Pyrazolen folgende N-Hydroxypyrazole hergestellt:

| | Hydroxypyrazol | Ausbeute [% der Theorie] | Festpunkt [°C] |
|---|---|---|---|
| b) | 4-Chlor-N-hydroxypyrazol | 58 | 122 |
| c) | 4-Jod-N-hydroxypyrazol | 27 | 125 |
| d) | 3,5-Dimethyl-N-hydroxypyrazol | 25 | 155 |

### Beispiel 2

a) 27,2 g (0,4 Mol) Pyrazol wurden in 300 ml Toluol gelöst. Unter Rühren wurden 25,4 g (0,43 Mol) KOH-Pulver (88 %ig) zugegeben und die Mischung auf 20°C gekühlt. Nach Zugabe von 0,3 g (0,013 Mol) Benzyltriethylammoniumchlorid wurden portionsweise 24,5 g (0,075 Mol) Dibenzoylperoxid (stabilisiert mit 25 % Wasser) zugegeben, wobei die Temperatur im Reaktionskolben auf 20°C gehalten wurde. Nach beendeter Zugabe wurde noch 2 Stunden gerührt, danach 100 ml Wasser zugegeben und die Phasen getrennt. Die wäßrige Phase wurde mit Schwefelsäure angesäuert und die dabei ausgefallene Benzoesäure abfiltriert. Das Filtrat wurde bei schwach saurem pH-Wert 3 mal mit je 100 ml Essigsäureethylester extrahiert und die vereinigten organischen Phasen wurden mit Natriumsulfat getrocknet. Das Lösungsmittel wurde anschließend im Rotationsverdampfer entfernt.
   Das zurückbleibende ölige Rohprodukt wurde analog Beispiel 1 aufgearbeitet.
   Ausbeute: 4,2 g (entsprechend 75 % der Theorie, bezogen auf umgesetztes Pyrazol).

In analoger Weise wurden aus entsprechend substituierten Pyrazolen folgende N-Hydroxypyrazole hergestellt:

| | Hydroxypyrazol | Ausbeute [% der Theorie] | Festpunkt [°C] |
|---|---|---|---|
| b) | 4-Chlor-N-hydroxypyrazol | 65 | 122 |
| c) | 4-Jod-N-hydroxypyrazol | 20 | 125 |

## Patentansprüche

1. Verfahren zur Herstellung von N-Hydroxypyrazolen der allgemeinen Formel I 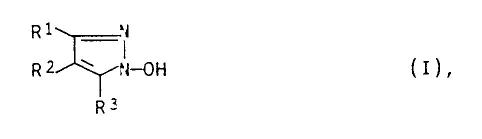 in der
R¹, R² und R³ gleich oder verschieden sein können und Wasserstoff oder einen Alkyl-, Halogen- oder Arylrest bedeuten können, oder die Substituenten
R¹ und R² oder R² und R³ zusammen mit den diese Reste tragenden Kohlenstoffatomen einen aromatischen oder nichtaromatischen Ring mit 5 oder 6 C-Atomen bedeuten,
dadurch gekennzeichnet, daß man
A) Pyrazole der allgemeinen Formel II 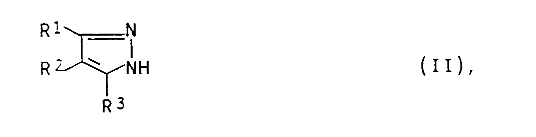 in der
die Reste R¹, R² und R³ die in Formel I angegebene Bedeutung haben, in an sich bekannter Weise mit einem Alkalihydroxid, Alkalihydrid oder Alkalicarbonat bei Temperaturen von 0 bis 50°C in ihre Metallsalze der allgemeinen Formel III 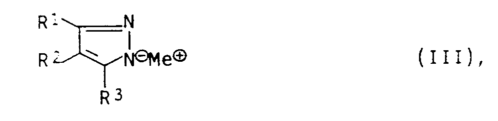 in der
die Reste R¹, R² und R³ die in Formel I genannten Bedeutungen haben und
Me^{⊕} ein Kation der Alkalimetalle bedeutet, überführt, und
B) die erhaltenen Metallsalze, der allgemeinen Formel III so mit Diacylperoxiden umsetzt, daß die Reaktionstemperatur zwischen 0 und 60°C liegt.

2. Verfahren gemäß Anspruch 1, dadurch gekennzeichnet, daß man als Diacylperoxid Dibenzoylperoxid verwendet.

3. Verfahren gemäß Anspruch 2, dadurch gekennzeichnet, daß man
A) die Pyrazole der allgemeinen Formel II in einem inerten Lösungsmittel in ihre Metallsalze der allgemeinen Formel III überführt und
B) zu der erhaltenen Lösung bzw. Suspensionen der Metallsalze der allgemeinen Formel III eine Lösung von trockenem Dibenzoylperoxid in einem inerten Lösungsmittel in der Weise zugibt, daß die Reaktionstemperatur zwischen 0 und 60°C liegt.

4. Verfahren gemäß Anspruch 2, dadurch gekennzeichnet, daß man Metallsalze der allgemeinen Formel III in Gegenwart von etwa äquimolaren Mengen Wasser mit dem Dibenzoylperoxid umsetzt.

5. Verfahren gemäß Anspruch 2, dadurch gekennzeichnet, daß man
A) die Pyrazole der allgemeinen Formel II in Wasser in an sich bekannter Weise mit einem Alkalihydroxid oder Alkalicarbonat in Lösungen oder Suspensionen ihrer Metallsalze der allgemeinen Formel III überführt und
B) die erhaltenen Metallsalze der Formel III bzw. ihre Lösungen oder Suspensionen in Wasser, in einem 2-Phasensystem aus Wasser und einem inerten, mit Wasser nicht oder nur schlecht mischbaren, organischen Lösungsmittel in Gegenwart eines geeigneten Phasentransferkatalysators mit Dibenzoylperoxid umsetzt.

6. Verfahren gemäß Anspruch 5, dadurch gekennzeichnet, daß man als Phasentransferkatalysator ein quartäres Ammoniumsalz oder -hydroxid in Mengen von 0,1 bis 1 Mol pro Mol Pyrazol einsetzt.

7. Verfahren gemäß Anspruch 5, dadurch gekennzeichnet, daß man handelsübliches, mit einer größeren Menge Wasser phlegmatisiertes Dibenzoylperoxid in festem Zustand bei Raumtemperatur zu einem 2-Phasensystem hergestellt aus Wasser, einem Alkalihydroxid, einem Pyrazol der Formel II, einem Phasentransferkatalysator und einem inerten mit Wasser nicht oder nur schlecht mischbaren, organischen Lösungsmittel zufügt.

8. Verfahren gemäß Anspruch 5, dadurch gekennzeichnet, daß man handelsübliches Dibenzoylperoxid in einem 2-Phasensystem bestehend aus Wasser, einem Pyrazol der Formel II, einem Phasentransferkatalysator und einem inerten, mit Wasser nicht oder nur schlecht mischbaren, organischen Lösungsmittel vorlegt und die Umsetzung durch Zugabe eines Alkalihydroxids ermöglicht.

## Claims

1. A process for the preparation of an N-hydroxypyrazole of the formula I 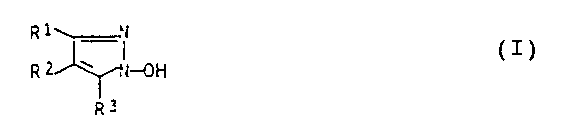 where R¹, R² and R³ may be identical or different and are each hydrogen, alkyl, halogen or aryl, or R¹ and R² or R² and R³ together with the carbon atoms carrying these radicals form an aromatic or nonaromatic ring of 5 or 6 carbon atoms, which comprises
A) converting a pyrazole of the formula II 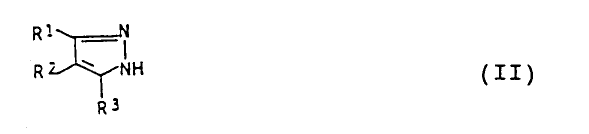 where R¹, R² and R³ have the meanings stated for formula I, at from 0 to 50°C in a conventional manner with an alkali metal hydroxide, alkali metal hydride or alkali metal carbonate into one of its metal salts of the formula III 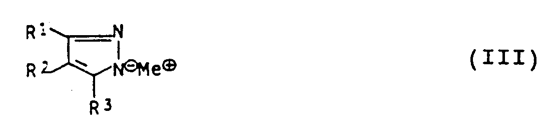 where R¹, R² and R³ have the meanings stated for formula I and Me^{⊕} is a cation of an alkali metal, and
B) the resulting metal salt of the formula III reacting with a diacyl peroxide in such a manner that the reaction temperature is from 0 to 60°C.

2. A process as claimed in claim 1, wherein the diacyl peroxide used is dibenzoyl peroxide.

3. A process as claimed in claim 2, wherein
A) a pyrazole of the formula II in an inert solvent is converted into one of its metal salts of the formula III and
B) a solution of dry dibenzoyl peroxide in an inert solvent is added to the resulting solution or suspension of the metal salt of the formula III in such a way that the reaction temperature is from 0 to 60°C.

4. A process as claimed in claim 2, wherein the metal salt of the formula III is reacted with the dibenzoyl peroxide in the presence of a roughly equimolar amount of water.

5. A process as claimed in claim 2, wherein
A) a pyrazole of the formula II in water is converted in a conventional manner with an alkali metal hydroxide or alkali metal carbonate into a solution or suspension of one of its metal salts of the formula III and
B) the resulting metal salt of the formula III or its solution or suspension in water is reacted with dibenzoyl peroxide in the presence of a suitable phase transfer catalyst in a 2-phase system consisting of water and an inert organic solvent which is water-immiscible or only slightly miscible with water.

6. A process as claimed in claim 5, wherein the phase transfer catalyst used is a quaternary ammonium salt or hydroxide in an amount of from 0.1 to 1 mole per mole of pyrazole.

7. A process as claimed in claim 5, wherein commercial dibenzoyl peroxide in the solid state, desensitized with a relatively large amount of water, is added at room temperature to a 2-phase system prepared from water, an alkali metal hydroxide, a pyrazole of the formula II, a phase transfer catalyst and an inert organic solvent which is water-immiscible or only slightly miscible with water.

8. A process as claimed in claim 5, wherein commercial dibenzoyl peroxide in a 2-phase system consisting of water, a pyrazole of the formula II, a phase transfer catalyst and an inert organic solvent which is water-immiscible or only slightly miscible with water is initially taken and the reaction is initiated by adding an alkali metal hydroxide.

## Revendications

1. Procédé de préparation de N-hydroxypyrazoles de la formule générale I 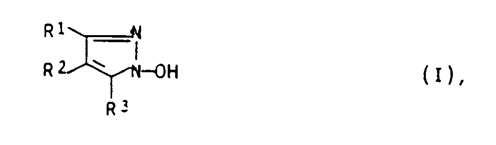 dans laquelle
R¹, R² et R³ peuvent être identiques ou différents et représentent chacun , indépendamment l'une de l'autre, un atome d'hydrogène ou un atome d'halogène, ou un radical alkyle ou aryle, ou bien les symboles
R¹ et R² ou R² et R³ forment ensemble avec les atomes de carbone qui portent ces restes un noyau aromatique ou non aromatique comportant 5 ou 6 atomes de carbone,
caractérisé en ce que
A) on convertit des pyrazoles de la formule générale II 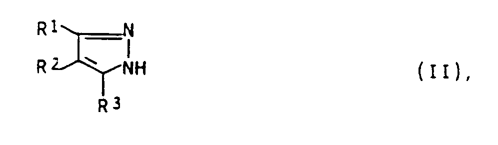 dans laquelle
les symboles R¹, R² et R³ ont les significations qui leur ont été attribuées à propos de la définition de la formule I, de manière en soi connue et à l'aide d'un hydroxyde de métal alcalin, d'un hydrure de métal alcalin ou d'un carbonate de métal alcalin, à des températures de 0 à 50°C, en leurs sels de métaux de la formule générale III 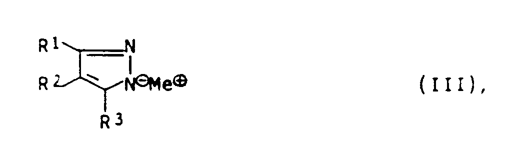 dans laquelle
les symboles R¹, R² et R³ ont les significations qui leur ont été attribuées à propos de la définition de la formule I et
Me^{⊕} représente un cation des métaux alcalins et
B) on fait réagir les sels de métaux ainsi obtenus, de la formule III, avec des peroxydes de diacyle en une manière telle que la température réactionnelle se situe entre 0 et 60°C.

2. Procédé suivant la revendication 1, caractérisé en ce que l'on utilise le peroxyde de dibenzoyle à titre de peroxyde de diacyle.

3. Procédé suivant la revendication 2, caractérisé en ce que
A) on convertit les pyrazoles de la formule II en leurs sels de métaux de la formule générale III dans un solvant inerte et
B) on ajoute une solution de peroxyde de dibenzoyle sec dans un solvant inerte aux suspensions ou solutions des sels de métaux de la formule générale III ainsi obtenues, en une manière telle que la température réactionnelle se situe entre 0 et 60°C.

4. Procédé suivant la revendication 2, caractérisé en ce que l'on fait réagir les sels de métaux de la formule générale III avec le peroxyde de dibenzoyle en présence de proportions environ équimolaires d'eau.

5. Procédé suivant la revendication 2, caractérisé en ce que
A) on convertit les pyrazoles de la formule générale II dans de l'eau, de manière en soi connue, à l'aide d'un hydroxyde de métal alcalin ou d'un carbonate de métal alcalin, en solutions ou suspensions de leurs sels de métaux de la formule générale III et
B) on fait réagir les sels de métaux de la formule III ainsi obtenus ou leurs solutions ou suspensions dans de l'eau avec le peroxyde de dibenzoyle, dans un système à deux phases constitué d'eau et d'un solvant organique inerte, non miscible ou seulement très difficilement miscible à l'eau, en présence d'un catalyseur de transfert de phases convenable.

6. Procédé suivant la revendication 6, caractérisé en ce qu'à titre de catalyseur de transfert de phases, on utilise un hydroxyde ou un sel d'ammonium quaternaire, en proportions de 0,1 à 1 mole par mole de pyrazole.

7. Procédé suivant la revendications 5, caractérisé en ce que l'on ajoute du peroxyde de dibenzoyle phlegmatisé avec une grosse proportion d'eau, usuel du commerce, à l'état solide, à la température ambiante, à un système à deux phases préparé à partir d'eau, d'un hydroxyde de métal alcalin, d'un pyrazole de la formule II, d'un catalyseur de transfert de phases et d'un solvant organique, inerte, non miscible ou seulement très difficilement miscible à l'eau.

8. Procédé suivant la revendication 5, caractérisé en ce que l'on introduit au préalable du peroxyde de dibenzoyle usuel du commerce dans un système à deux phases constitué d'eau, d'un pyrazole de la formule II, d'un catalyseur de transfert de phases et d'un solvant organique, inerte, non miscible ou seulement très difficilement miscible à l'eau et on rend la réaction possible par l'addition d'un hydroxyde de métal alcalin.
